# EUROPEAN PATENT SPECIFICATION

(11) **EP 2 729 188 B1**
(45) Date of publication and mention of the grant of the patent: **04.11.2020**
(21) Application number: 12737665.5
(22) Date of filing: 06.07.2012
(51) Int. Cl.: A61L 9/14, A01N 25/06, B65D 83/14, B05B 5/047, A61K 9/00, A01N 59/00, A01M 7/00

(54) **COMPRESSED GAS AEROSOL DISPENSER WITH ENHANCED INTENSITY AND LONGEVITY OF ACTIVES**
DRUCKGASAEROSOLSPENDER MIT ERHÖHTER INTENSITÄT UND LEBENSDAUER VON WIRKSTOFFEN
DISTRIBUTEUR D'AÉROSOLS À GAZ COMPRIMÉ AYANT UNE INTENSITÉ ET LONGÉVITÉ DES PRINCIPES ACTIFS AMÉLIORÉES

(30) Priority: 08.07.2011 US 201161457925 P
(43) Date of publication of application: 14.05.2014
(73) Proprietor: S.C.JOHNSON & SON, INC., Racine, WI 53403-2236 (US)
(72) Inventor: NGUYEN, Peter, N., Racine, WI 53402 (US); SHAH, Bhaveshkumar, Kenosha, WI 53142 (US)
(74) Representative: Murgitroyd & Company
(86) International application number: PCT/US2012/045730
(87) International publication number: WO 2013/009607

(56) References cited:
- EP-A1- 1 593 396
- WO-A1-99/21659
- WO-A1-2004/062813
- WO-A1-2007/002778
- WO-A1-2011/159350
- WO-A2-02/089862

## Description

### FIELD OF INVENTION

The invention is directed to improving or enhancing intensity and longevity of active component(s) of an aerosol composition, in particular where the active component is one or more of a fragrance, odor elimination compound, insecticide, disinfectant or antimicrobial, and the aerosol composition is propelled by a compressed gas propellant.

### BACKGROUND OF THE INVENTION

Aerosol products for dispensing fragrances and odor treating agents are known in the art which include a compressed gas propellant, see e.g. WO99/21659, WO2004/062813 A1, EP1593396 A1 or WO2007/002778 A1. Whether for applying to the air or a fabric or other surface, or treating air or fabric or other surface to eliminate an odor therein or thereon, it is desirable to provide the active component of the treating composition at a certain level or intensity over a length of time that allows the benefits to be appreciated for an extended period of time. Prior to the present invention, it had been believed that larger aerosol particle sizes of a fragrance or odor treating composition were not desirable or as advantageous as small particle sizes because the particles of composition would fall out of the air faster and result in wetting of the surfaces upon which the particles fall. Since higher spray rates generally provide larger particle sizes, higher spray rates were also considered not to be desirable. It would also be advantageous to provide good intensity and fall out with a low level of or without the inclusion of an alcohol (which in the past has been included to aid in evaporation) in a composition since alcohol undesirably results in a higher volatile organic content (VOC) to the composition.

### SUMMARY OF THE INVENTION

An active(s)-containing aerosol composition, most preferably an odor treating or fragrance freshening aerosol composition, is provided having improved intensity of the active(s) over an extended period of time. The composition is propelled for dispensing from a suitable container by a compressed gas propellant. The improvement experienced by a consumer of enhanced intensity of one or more actives in an environment of use is in terms of at least freshness, longer lasting, and consistency of quality or character between initial and terminal use, which each provide for a greater product impact on the user of the composition or product.

The invention allows for the use of any conventional or known active, such as to provide an air or fabric or other surface fragrance or odor treatment, insecticidal, disinfecting or antimicrobial composition, and yet provides enhancement of the active's properties, e.g., fragrance and/or odor elimination, with these conventional actives. This is achieved by maintaining or controlling certain properties of the composition present during dispensing of the product. The composition is preferably single phase and has a low to no voc. To provide a single phase composition, a surfactant or suitable non-water co-solvent will be included in the composition. Further, while an alcohol may be present, such is not required to be included. The composition includes as essential components a compressed gas propellant, at least one active ingredient (e.g., a fragrance, odor elimination component, insecticide, disinfectant, antimicrobial or mixtures thereof), and water as a solvent carrier. The composition may optionally also include one or more surfactant, buffering agent, corrosion inhibitor, pH adjuster, preservative and other adjuvants known for inclusion in such compositions. Properties of the compositions which are controlled to provide the enhanced intensity and longevity of the active ingredient(s) in particular include the spray rate of the composition, and the average particle size of the composition. Additional properties to aid in the enhancement of intensity and longevity are initial and terminal pressures under which the composition is present in a dispenser, and the pressure drop ratio. The described combination of properties are selected, maintained or controlled to provide an optimum dispensing over the life of the product so as to obtain more active(s) in the air, prior to fall out to a surface, to provide greater intensity over time of the composition active(s). For example, consumers usually spray an air, fabric or other surface treating aerosol product about on average of 1-7 seconds depending on intended use, e.g., whether treating air, hard surface or soft surface. With each spray, not only is the total product being delivered important, but also the particle size of the sprayed composition, as it plays an important role in the effectiveness of and the consumer's experience of the active(s). The combination of particle size and spray rate has been determined to have an effect on experiencing the active(s), e.g., fragrance.

The spray rate of the active(s)-containing composition is selected and controlled to be greater than about 1.5 grams per second (g/s) to about 3.0 g/s, preferably about 1.6 to about 2.5 g/s. The particle size of the active(s)-containing composition is concurrently selected and controlled to be in a range of about 60 to about 100 microns, preferably about 60 to about 90 microns. When the spray rate has been within the desired range, e.g., 2.0 g/s, but the particle size has been larger than the desired range, e.g., 145-200 microns, a noticeable lesser intensity in the active ingredient, e.g., a fragrance, was found to occur consistently over a period of time. Accordingly, the invention provides at the beginning of the life of the product and through to the end of the life of the product optimal performance of the active(s) through intensity over time, thereby achieving consistency of quality or character of the product over the life of the product. The parameters of the invention provide an optimal performance of product over an extended period of time. The particle size of the composition is such that, in combination with the spray rate, the product does not get easily carried away in air flow or immediately fall to the surface. It is present over a sufficient duration of time to give immediate and continuing performance.

### BRIEF DESCRIPTION OF DRAWING

Figure 1 is a graph illustrating test results as described below of fragrance intensity over time based on different spray rates and particle sizes.
Figure 2 is a graph of a control or benchmark composition illustrating test results as described below of fragrance intensity over time.
Figure 3 is a graph showing additional test results as described below of fragrance intensity over time.

### DETAILED DESCRIPTION OF THE INVENTION

The aerosol active(s)-containing compositions have a general formulation which includes water as a solvent carrier in an amount for the water to serve as a major component of the composition, at least one compressed gas propellant, and at least one active ingredient, such as fragrance(s), odor eliminating compound(s), insecticide(s), disinfectant(s) and antimicrobial(s). For ease of discussion, the invention will be described in terms of the active(s) being one or more of a fragrance or odor eliminating compound, which are also part of preferred embodiments of the invention. The composition is preferably single phase and includes one or more actives in an amount in a range of about 0.1 to about 2.5 weight percent (wt.%) based on 100 wt.% being the total composition. The composition can include additional components such as surfactant(s), co-solvent(s), buffering agent(s), corrosion inhibitor(s), pH adjuster(s), and conventional adjuvants as known by one skilled in the art based on the intended use of the composition. Water is the primary solvent. Alcohols can be included in the composition as a co-solvent, however, such are not desirable for inclusion as a co-solvent since such raises the VOC of the composition and the advantages of the invention are obtained in the absence of an alcohol compound. The components of the composition can be present in amounts suitable for their intended use based on the selected active(s) in the aerosol composition.

When a co-solvent is included, preferably the co-solvent is selected and present in an amount to provide a low or zero volatile organic content (VOC). Most preferably, the VOC is zero, but a co-solvent can be present in a low amount.

The enhanced properties of the composition are provided by a combination of at least
(1) Spray rate for dispensing of the composition which is greater than about 1.5 to about 3.0 grams/second (g/s), preferably about 1.6 to about 2.5 g/s and further about 1.7 to about 2.2 g/s, wherein the spray rate is determined by measuring the rate of product expelled by an initial spray of product for a 60 second duration of a 100% full container of product (this spray duration resulting in dispensing of approximately one half of a conventional 120 ml aerosol container); and
(2) Particle size of the composition during dispensing is maintained to provide an average particle size of about 60 to about 100 microns, preferably about 60 to about 90 microns and more preferably about 60 to about 80 microns. Particle size was measured using a particle analyzer as manufactured by Malvern Instruments, Inc., Model STP5311, which is described as the Short Bench Spraytec Laboratory system with 950 mm optical bench and 300 mm lens. Samples are spray tested according to the programmed software of the Malvern particle analyzer to obtain mass median diameter (MMD) for a particle diameter in which 50% of the volume sampled is below the measured particle diameter. The measured results are provided for 10 seconds and 60 seconds.

The additional properties of pressure within the container, and the ratio of pressure drop also assist in improving the intensity of the active(s) of the composition over time. More particularly, the product in a dispensing container is under an initial or starting pressure in a range of from about 6.89 to about 12.41 bar (about 100 to about 180 psig) at 70°F (21°C) (all pressure measurements herein being understood to be at 70°F (21°C)) to a terminal pressure (i.e., less than 10% of product remaining in the dispensing container) from about 2.07 to about 6.21 bar (about 30 to about 90 psig). Preferably, starting pressure is about 8.27 to 11.03 bar (about 120 to 160 psig) and terminal pressure is about 2.76 to 4.14 bar (about 40 to 60 psig). Further, having a greater initial headspace will keep the terminal pressure higher. An optimal headspace is at least or greater than about 35% which provides for less spray rate variation.

An advantageous pressure drop ratio is provided, corresponding to head space of 25% and 50% by volume of about less than 4:1 for a headspace of 25% by volume and a pressure drop ratio of 2:1 for a headspace of 50% by volume.

The spray rate range of (1) above ensures an ideal amount of active(s) is(are) released from the container. The active(s)-containing compressed gas composition is provided in a dispensing container for storing and dispensing the composition as an aerosol spray. The ratio of compressed gas to composition is about 50:50 to about 25:75 by volume. The compressed gas can be compressed air, nitrogen, nitrous oxide, an inert gas, carbon dioxide, or combinations thereof. The active(s)-containing composition is to be released at a spray rate of greater than about 1.5 g/s to about 3.0 g/s, wherein the spray rate is determined as set forth above in (1), i.e., 60 second expulsion from a 100% full container. The average particle size of the spray is about 60 to about 100 microns, preferably about 60 to about 90 microns, and more preferably about 60 to about 80 microns. The active component of the composition can be one or more conventional natural or synthetic perfuming or fragrance compound, an odor elimination compound, such as triethylene glycol (TEG), an insecticide, a disinfectant, an antimicrobial, or the like.

Provision of the described particle size in combination with the described spray rate results in enhanced fragrancing and odor elimination, when the active is a fragrance or odor eliminator, upon dispersal of the fragrance and/or odor treating composition. The size of the particles allows for longer residence time in the treated air. The longer residence time in the air improves the fragrance experience of the consumer with respect to maintaining the character of the fragrance over a longer period of time.

The maintaining or control of the initial and terminal pressure within the ranges described above ensures sufficient breakup of the liquid fragrance or odor treating composition as it is dispensed from a container. Particle size of the aerosol can be affected by the pressure of the aerosol.

The maintaining of the pressure drop as described above to be less than 4:1 when the head space is 25% by volume or 2:1 when the headspace is 50% by volume leads to a more consistent spray, in particular as to particle size and spray rate. A more consistent spray enhances the fragrance and odor elimination experience, i.e., the intensity experienced by the consumer over the life of the product.

Combination of the features of spray rate and average particle size serve to enhance the effects of the composition as experienced by the consumer. Further combination of these features with pressure and pressure drop ratio control as described above serves to further increase the benefits of the composition upon spraying into an environment of use.

While the invention is described primarily with respect to compositions for treating air, e.g., fragrancing and/or odor elimination, the invention is also applicable to fragrancing fabric or other surfaces or eliminating odors on fabric or other surfaces. Compressed gas aerosols are advantageous in treating fabrics since such provide for a good evaporation rate from fabric and avoid resoiling following treatment based on the ability to be provided with an overall smaller particle size range and distribution as compared generally, for example, to manual pump sprayers. The evaporation rate results in a longer life of the product in use.

Aerosol compositions useful in the invention for enhancing the intensity of the active component over time have the essential components of at least one compressed gas propellant, at least one active ingredient, and water as a solvent carrier, wherein the water is present in an amount so that the water serves as a major component of the composition. The active(s) can be one or more of a fragrance, odor elimination component, insecticide, disinfectant, and antimicrobial. Additional components can be present in the composition such as for example, one or more of a surfactant, buffering agent, pH adjuster, co-solvent, corrosion inhibitor, preservative, and other known adjuvants.

As to air or fabric treatment compositions, such is generally preferred as set forth in Table 1 below. The compositions are present as a single phase composition.

**TABLE 1**

| Ingredients | Wt.% Range |
|---|---|
| Water | about 80 to about 99 |
| Surfactant(s) | about 0.25 to about 2 |
| Active(s) | about 0.1 to about 2.5 |
| Compressed Gas Propellant(s) | about 0.25 to about 2 |
| pH Adjuster(s) | optionally present in an amount sufficient to achieve a pH of about 4 to about 10 |
| Corrosion Inhibitor(s) | 0 to about 5 |
| Non-Water Solvent(s) | 0 to about 10 |
| Buffer (s) | 0 to about 5 |
| Preservative(s) | 0 to about 1 |

Weight percent (wt.%) of the total composition in Table 1 and as used in the description and claims is based on 100 wt.%. The ingredient wt.% given is based on the wt.% of the whole ingredient and not simply on the active(s) of the ingredient.

The water component is a solvent carrier and can be deionized water, reverse osmosis water, distilled water, tap water, and/or the like. Preferred are deionized water and reverse osmosis water. Generally, water is present in an amount greater than about 80 wt.% but less than 100 wt.%. The preferred amount of water present is as set forth in Table 1 above. More preferably, water is present in an amount of about 90 to about 99 wt.%, and most preferably in an amount of about 92 to about 97.5 wt.%.

Surfactants suitable for inclusion in the formulation generally can be nonionic, cationic, anionic, amphoteric, zwitterionic, or mixtures thereof. The surfactant will be generally selected in view of the dispensing container used. For example, a composition stored and dispensed from a steel or steel alloy-based container preferably includes a nonionic and/or amphoteric surfactant (which are less corrosive) whereas an aluminum or plastic container on the other hand can include those and/or other surfactants. The surfactant component can be one or more surfactants.

Suitable nonionic surfactants useful in the aerosol composition include, but are not limited to, polyalkoxylated hydrogenated castor oil, preferably polyethoxylated hydrogenated caster oil such as TAGAT CH60 (60 ethylene oxide (EO) units), TAGAT CH40 (40 EO units); hydrogenated and ethoxylated castor oil blends, e.g. EUMULGIN HPS (40 EO units); secondary alcohol ethoxylates, e.g., TERGITOL brand surfactants such as TERGITOL 15-S-12 and TERGITOL 15-S-7; ethoxylated linear alcohols, e.g., LUTENSOL brand such as LUTENSOL A08 (8 EO units); sorbitan monooleate; polyethylene sorbitan monooleate; polyoxyethylene sorbitan monolaurate; alkyl polyglycosides; polyethyleneoxide/polypropyleneoxide; alkyl phenol ethoxylated carboxylated alcohols; and mixtures thereof.

The at least one nonionic surfactant is present in an amount range preferably as set forth in Table 1, i.e., in a range of about 0.1 to about 2.0 wt.%, and more preferably in a range of about 0.5 to about 1.0 wt.%, and most preferably about 0.5 to about 0.8 wt.%.

Cationic surfactants suitable for inclusion in the aerosol composition include, but are not limited to, the following: quaternary ammonium salts, polyoxyethylene alkyl, alicyclic amines, and mixtures thereof.

The at least one cationic surfactant is present in an amount range of preferably 0 to about 2.0 wt.%, and more preferably present in an amount of 0 to about 1.0 wt.%. As set forth above, the cationic surfactant is preferably used in combination with a nonionic surfactant and not as the sole surfactant.

Fragrance(s) suitable for inclusion as an active in the aerosol composition can be a natural or synthetic fragrance, based on a single component or a blend of components. Fragrances are commercially available from various fragrance manufacturers, such as Takasago, International Flavors and Fragrances, Inc., Quest, Firmenich, Givaudan, Symrise, and the like. Other actives useful are as known to one skilled in the art based on the intended use of a composition containing the selected active(s).

The fragrance(s) is(are) present in an amount range generally as set forth above in Table 1 and is(are) preferably present in a range of about 0.1 to about 2.0 wt.%, and more preferably present in a range of from about 0.3 to about 1.0 wt.%.

The compressed gas propellant may be any suitable conventionally known compressed gas propellant, including, but not limited to, nitrogen, an inert gas, air, nitrous oxide, carbon dioxide, or mixtures thereof.

The compressed gas propellant is present in an amount generally as set forth above in Table 1, i.e., is preferably present in a range of about 0.25 to about 2 wt.%, and more preferably in a range of about 0.5 to about 1.0 wt.%. The compressed gas propellant is pressurized in a range of from about 6.89 to about 12.41 bar (about 100 to about 180 psig) as an initial or starting pressure for a 100% full container. As the composition is expelled from the container the pressure drops. When less than 10% volume of the composition remains in the container, the pressure will be in the range of about 2.07 to about 6.21 bar (about 30 to about 90 psig).

Corrosion inhibitor(s) suitable for inclusion in the compressed gas air treatment formulation is(are), but not limited to, phosphates, such as potassium dihydrogen phosphate, potassium hydrogen phosphate, diammonium phosphate, potassium phosphate (monobasic or dibasic), sodium phosphate (monobasic or dibasic); nitrites, such as sodium nitrite, potassium nitrite, and ammonium nitrite; aminomethyl propanol; silicates, such as sodium meta-silicate; and/or amines.

The corrosion inhibitor(s) is(are) present in the general amount as set forth above in Table 1, and is(are) preferably present in a range of about 0.01 to about 1 wt.%, and more preferably in a range of about 0.1 to about 0.5 wt.%.

Non-water solvents or co-solvents suitable for use include glycols, alcohols, glycol ethers, ketones, and esters. Examples of glycol non-water solvents suitable for use include, but are not limited to, alkylene glycols, such as propylene glycol and triethylene glycol. When alcohols are present as a co-solvent, such is preferably present in a low amount. However, the inclusion of an alcohol is generally not desirable since such increases the VOC of the composition and the advantages of the invention are obtainable in the absence of an alcohol.

The non-water solvent(s) is(are) present in an amount as set forth above in Table 1, i.e., preferably is present in a range of 0 to about 10 wt.%, and more preferably in a range of about 0.1 to about 6.0 wt.%. Most preferably, the non-water solvent is present in an amount of less than or equal to 0.1 wt.% so that the formulation has a low or no VOC.

Additional compounds suitable for inclusion in the compressed gas composition as pH adjusters or controllers include, but are not limited to, carbonates, such as sodium carbonate; silicates, such as sodium meta-silicate pentahydrate (which may provide a dual function as a pH adjuster and corrosion inhibitor); phosphates, such as disodium phosphate, and dipotassium phosphate; hydroxides, such as sodium hydroxide; ammonium hydroxide; THAM-Tris-(hydroxymethyl) aminoethane; 2-amino-2-methylpropane diol; and the like.

The pH adjuster is optional. The pH adjuster when present is used in an amount sufficient to obtain a pH in a range of about 4 to about 10.

Buffer compound(s) suitable for inclusion in the aerosol composition includes, but is not limited to, bicarbonates, such as sodium bicarbonate; phosphates; ammonium hydroxide; THAM-Tris (hydroxymethyl) aminoethane; 2-amino-2-methyl-propane diol; and the like. It is noted that some well known pH buffering agents, such as phosphates, carbonates, ammonium hydroxide, THAM-Tris (hydroxymethyl) aminoethane, and 2-amino-2-methylpropane diol, will provide a multi-purpose function of corrosion inhibitor, pH adjustor, and buffering agent. In such instance, one or a combination of ingredients may be used to meet these functions and amounts thereof adjusted accordingly within the scope of the invention.

The buffer(s) is(are) present in an amount as generally set forth in Table 1 above, and preferably in a range of about 0.01 to about 1 wt.%, and more preferably in a range of about 0.1 to about 0.5 wt.%.

Preservative(s) suitable for inclusion in the aerosol composition include, but are not limited to isothiazolinones, such as 1,2,-benzisothiazole-3(2H)-one and 2-methylisothiazole-3(2H)-one, which is sold as a blend under the trade name ACTICIDE MB or ACTICIDE MBS; 2-methyl-4-isothiazolin-3-one, which is sold under the trade name NEOLONE M-10; and 2-methyl-2H-isothiazol-3-one and 3-iodo-2-propynyl-butyl carbamate, which is sold as a blend under the trade name ACTICIDE IM.

The preservative(s) is(are) present in an amount as generally set forth above in Table 1, preferably in an amount in the range of about 0.01 to about 1.0 wt.%, more preferably in a range of about 0.01 to about 0.5 wt.%, and most preferably in a range of about 0.05 to about 0.2 wt.%.

Dispensing containers suitable for use can be essentially any type of container that can hold a pressure and meet commercial safety standards. For example, conventional 2 piece and 3 piece aerosol containers of steel, steel alloy, aluminum, plastic or the like are suitable for use. Such may be lined or unlined. The steel or steel alloy containers may be tin-plated or not. An example of a suitable aerosol container is a 3-piece tin plated steel can lined with an epoxy-urea lining. Plastic containers may be made of PET (polyethylene terephthalate), polycarbonate, polyethylene, PEN (polyethylene naphthalate) and other polyesters and generally other plastics known for use. The dispensing actuator for the container will be selected based on the spray rate and particle size desired to be provided based on the invention described herein.

### Tests To Illustrate Sensory Advantages Of The Invention

In two sets of tests, fragrance-containing aerosol compositions of the invention, comparative compositions and a benchmark or control composition were tested to show the improvement in fragrance intensity over time of the inventive compositions.

The tests conducted were sensory in nature and involved a test period from 0 to 180 minutes (3 hours) and an Intensity Rating scale using a universal scale of 0 to 15 wherein 0 indicates no odor, 1-2 = trace, 3-5 = slight, 6-8 = moderate, 9-12 = strong and 13-15 = extreme. A desired Intensity Rating for a fragrance-containing composition based on the 0-15 universal scale is 10 to 2, preferably 8 to 3. The Intensity Rating for compositions containing one or more actives other than a fragrance will need to be based on a fragrance present therewith, since certain actives will have no smell, or should have no smell in use, in order to judge intensity based on a sensory evaluation as described herein.

In the first set of tests as described here, Test Examples (1)-(3) are of the invention, Test Example (4) is a comparative test, and Test Example (5) is a benchmark or control test. Inventive Test Examples (1)-(3) and Comparative Test Example (4) were identical fragrance aerosol compositions for dispensing into the air. The formula for the composition of Benchmark or Control Test Example (5) is set forth below. Test Examples (1)-(5) involved spray rates and particle sizes as follows:
(1) Target spray rate: 1.6 g/s
   Average Particle Size: 60-80 microns
(2) Target spray rate: 1.8 g/s
   Average Particle Size: 60-80 microns
(3) Target spray rate: 2.5 g/s
   Average Particle Size: 60-80 microns
(4) Target spray rate: 2.0 g/s
   Average Particle Size: 145-200 microns
(5) Target spray rate: 2.0 g/s
   Average Particle Size: 60-80 microns

The composition formula for Test Example (5), which is the benchmark or control composition, is shown below in Table 2.

**Table 2**

| **Ingredients** | **Wt.%** |
|---|---|
| Deionized Water | 97.859 |
| Sodium Nitrite | 0.097 |
| Morpholine | 0.048 |
| TAGAT CH60¹ | 0.468 |
| TERGITOL 15-S-7² | 0.281 |
| Fragrance | 0.100 |
| Propylene Glycol | 0.468 |
| Nitrogen | 0.678 |
| TOTAL | 100.000 |
| ¹ - PEG-60 Hydrogenated Castor Oil, a nonionic surfactant | |
| ² - Secondary Alcohol Ethoxylate, a nonionic surfactant | |

Test Examples (1)-(5) were each evaluated by 15 trained sensory panelists. The test protocol utilized is further described below. Each of Test Examples (1)-(5) were tested under a pressure of 9.65-10.00 bar (140-145 psig). Test Example (5) is present as a control example so that upon recreation of the tests, such serves as a benchmark to insure that results are comparable as between test samples, for example to gauge the valuation standard of the Intensity Rating scale. The component make-up of Test Example (5) is unrelated to the inventive compositions described herein.

The Intensity Ratings provided by the sensory panelists for the tests for each of Test Examples (1)-(5) are set forth below in Table 3.

**Table 3**

| **Test Example** | **5 Min. Averages** | **30 Min. Averages** | **60 Min. Averages** | **90 Min. Averages** | **120 Min. Averages** | **150 Min. Averages** | **180 Min. Averages** |
|---|---|---|---|---|---|---|---|
| (1) | 5.85 | 5.16 | 4.21 | 3.71 | 3.01 | 2.56 | 1.73 |
| (2) | 5.84 | 5.07 | 4.42 | 3.61 | 3.07 | 2.81 | 2.01 |
| (3) | 5.78 | 5.37 | 4.75 | 4.31 | 3.84 | 3.19 | 2.40 |
| (4) | 4.63 | 4.43 | 3.72 | 3.33 | 2.39 | 2.13 | 1.55 |
| (5) | 5.64 | 4.99 | 4.28 | 3.42 | 2.98 | 2.05 | 1.55 |

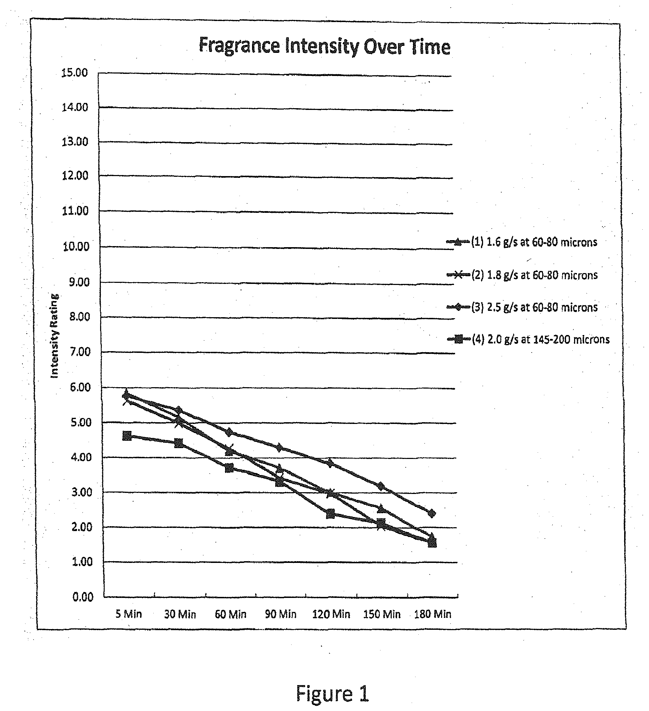

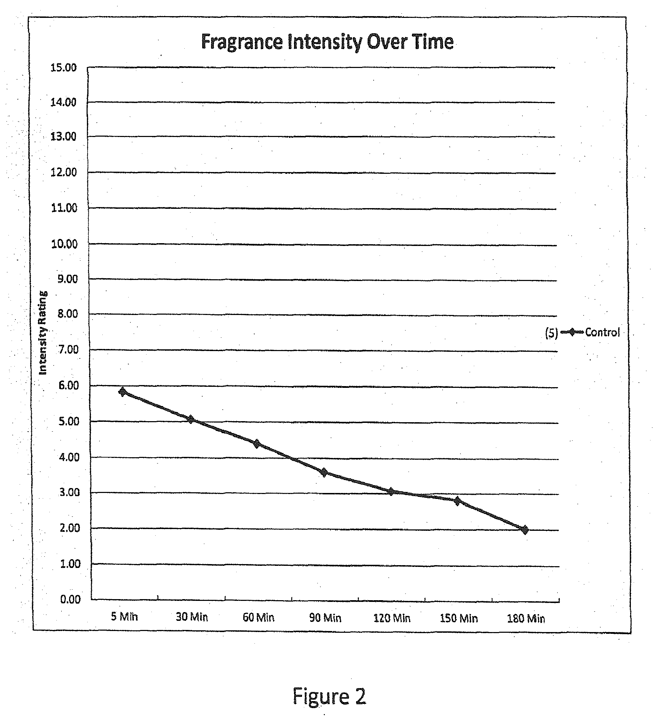

These results are graphically shown in Figure 1 as to Test Examples (1)-(4) and in Figure 2 as to Test Example (5). As shown in Figure 1, the intensity of the Inventive Test Examples (1)-(3) is greater as the spray rate is increased, and Comparative Test Example (4) illustrates that even when at a spray rate within the invention, if the particle size is outside that of the invention, the intensity is lower as compared to the compositions of the invention.

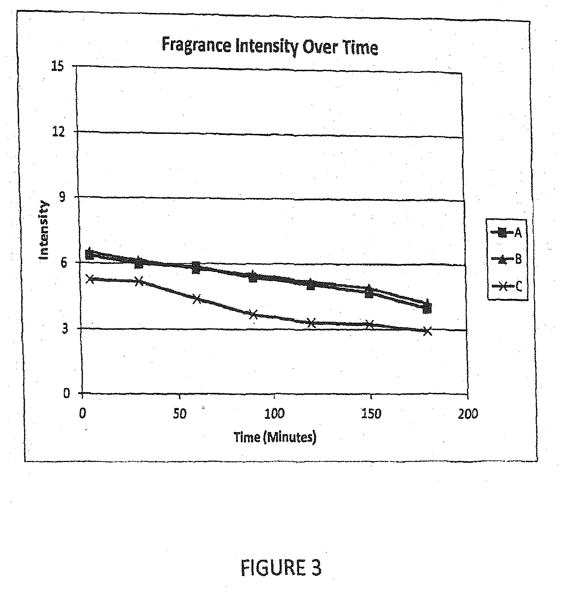

Figure 3 shows the results of the second set of comparative testing. Test Examples A, B. and C were of the same identical formulas as Test Examples (1)-(4) above. The spray rate, average particle size, and pressure of Test Examples A, B, and C were as follows:
(A) Target spray Rate: 2.5 g/s
   Average particle Size: 60-80 microns
   Pressure: 9.31 bar (135 psig)
(B) Target spray Rate: 3.0 g/s
   Average particle Size: 60-80 microns
   Pressure: 9.31 bar (135 psig)
(C) Target spray Rate: 2.0 g/s
   Average particle Size: 145-200 microns
   Pressure: 9.31 bar (135 psig)

The sensory ratings for Test Examples A-C are set forth below in Table 4.

**Table 4**

| **Test Example** | **5 Min. Averages** | **30 Min. Averages** | **60 Min. Averages** | **90 Min. Averages** | **120 Min. Averages** | **150 Min. Averages** | **180 Min. Averages** |
|---|---|---|---|---|---|---|---|
| A | 6.36 | 5.99 | 5.86 | 5.40 | 5.07 | 4.71 | 4.0 |
| B | 6.52 | 6.14 | 5.78 | 5.54 | 5.20 | 4.92 | 4.22 |
| C | 5.27 | 5.19 | 4.40 | 3.71 | 332 | 3.26 | 2.93 |

As shown by the sensory results, which are graphically illustrated in Figure 3, the inventive Test Examples A and B have a greater intensity than Comparative Test Example C.

### Test Protocol

The test protocol for fragrance Intensity Evaluation involved delivering approximately five (5) grams of a Test Example into an approximately 22.65 m³ (800 cubic foot) test room, i.e., 2.74 x 3.02 x 2.74 = 22.71 m³ (9' x 9.9' x 9' = 801.9 cu. ft.). Each room had low emitting finishes and HVAC system to mimic a home setting. The temperature of the room was maintained at 21°C. Fifteen sensory panelists trained as to the physical testing procedure and intensity rating based on the 15-point universal Intensity Rating scale described above, evaluated the fragrance delivered in the test room through a hatch into the test room as described below. The sensory panelists evaluated each product composition at 5, 30, 60, 90, 120, 150 and 180 minute intervals over a three hour period.

In filling the test room with the 5 grams of product, the person dispensing the Test Example product stands just inside the door to the test room to spray. Each test product is sprayed from the same place inside the door.

A hatch is provided into each test room. The sensory panelists provide their evaluations of the dispersed test product through the hatch. The door to the hatch will remain closed between individual panelists. A step stool is provided if necessary to ensure that each panelist can access the hatch on an approximate equivalent level for evaluation. The evaluation then occurs based on sensory perception by the panelist of the dispersed product and providing an intensity rating based on the 15-point Intensity Rating scale described above.

The exemplary embodiments herein disclosed are not intended to be exhaustive or to unnecessarily limit the scope of the invention. The exemplary embodiments were chosen and described in order to explain the principles of the present invention so that others skilled in the art may practice the invention. As will be apparent to one skilled in the art, various modifications can be made within the scope of the aforesaid description. Such modifications being within the ability of one skilled in the art

## Claims

1. An article for fragrance dispensing and/or odor treating comprising
(1) an aerosol air treatment composition comprising
(a) at least one compressed gas propellant;
(b) 0.1 to 2.5 wt.% of at least one active ingredient; and
(c) water as a solvent carrier and in an amount to serve as a major component of said composition,
wherein said compressed gas propellant is present in relation to said composition in a ratio of 50:50 to 25:75 by volume; and
(2) an aerosol dispensing container adapted to contain and dispense said composition including
(d) an initial pressure in said container of 6.89-12.41 bar (100-180 psig) at 21°C;
(e) a nozzle which dispenses said composition at a spray rate of greater than 1.5 g/s to 3.0 g/s and in an average particle size of 60 to 100 microns; and
(f) a pressure drop ratio over a dispensing life of said composition from said container of less than 4:1 when said container has a headspace of 25% by volume and of 2:1 when said container has a headspace of 50% by volume.

2. The article of claim 1, wherein said compressed gas is one or more of air, nitrogen, carbon dioxide, nitrous oxide, and an inert gas.

3. The article of claim 1, wherein said at least one active ingredient is one or more of a fragrance, an odor eliminating compound, an insecticide, an antimicrobial, and a disinfectant.

4. The article of claim 1, wherein said spray rate is from 1.6 g/s to 2.5 g/s.

5. The article of claim 1, wherein said average particle size is from 60 to 90 microns.

6. The article of claim 1, wherein said average particle size is from 60 to 80 microns.

## Patentansprüche

1. Ein Artikel zur Duftstoffabgabe und/oder zur Geruchsbehandlung, der Folgendes beinhaltet:
(1) eine Aerosolluftbehandlungszusammensetzung, die Folgendes beinhaltet:
(a) mindestens ein Druckgastreibmittel;
(b) zu 0,1 bis 2,5 Gew.-% mindestens einen aktiven Inhaltsstoff; und
(c) Wasser als einen Lösungsmittelträger und in einer Menge, um als eine Hauptkomponente der Zusammensetzung zu dienen,
wobei das Druckgastreibmittel in Bezug auf die Zusammensetzung in einem Volumenverhältnis von 50 : 50 bis 25 : 75 vorliegt; und
(2) einen Aerosolabgabebehälter, der angepasst ist, um die Zusammensetzung zu enthalten und abzugeben, der Folgendes umfasst:
(d) einen Anfangsdruck in dem Behälter von 6,89-12,41 Bar (100-180 psig) bei 21 °C;
(e) eine Düse, die die Zusammensetzung mit einer Sprührate von mehr als 1,5 g/s bis 3,0 g/s und mit einer durchschnittlichen Teilchengröße von 60 bis 100 Mikrometer abgibt; und
(f) ein Druckverlustverhältnis im Laufe eines Abgabelebens der Zusammensetzung aus dem Behälter von weniger als 4 : 1, wenn der Behälter einen Kopfraum von 25 Volumen-% aufweist, und von 2 : 1, wenn der Behälter einen Kopfraum von 50 Volumen-% aufweist.

2. Artikel gemäß Anspruch 1, wobei das Druckgas eines oder mehrere von Luft, Stickstoff, Kohlendioxid, Lachgas und einem Edelgas ist.

3. Artikel gemäß Anspruch 1, wobei der mindestens eine aktive Inhaltsstoff eines oder mehrere von einem Duftstoff, einer geruchsentfernenden Verbindung, einem Insektizid, einer antimikrobiellen Substanz und einem Desinfektionsmittel ist.

4. Artikel gemäß Anspruch 1, wobei die Sprührate von 1,6 g/s bis 2,5 g/s beträgt.

5. Artikel gemäß Anspruch 1, wobei die durchschnittliche Teilchengröße von 60 bis 90 Mikrometer beträgt.

6. Artikel gemäß Anspruch 1, wobei die durchschnittliche Teilchengröße von 60 bis 80 Mikrometer beträgt.

## Revendications

1. Un article pour distribuer un parfum et/ou traiter des odeurs comprenant
(1) une composition de traitement de l'air par aérosol comprenant
(a) au moins un gaz propulseur comprimé ;
(b) de 0,1 à 2,5 % en poids d'au moins un ingrédient actif ; et
(c) de l'eau en tant que véhicule de solvant et en une quantité faisant office de composant principal de ladite composition,
dans lequel ledit gaz propulseur comprimé est présent relativement à ladite composition dans un rapport de 50/50 à 25/75 en volume ; et
(2) un conteneur de distribution d'aérosol conçu pour contenir et distribuer ladite composition incluant
(d) une pression initiale dans ledit conteneur de 6,89 à 12,41 bar (100 à 180 psig) à 21 °C ;
(e) une buse qui distribue ladite composition à une vitesse de pulvérisation supérieure à de 1,5 g/s à 3,0 g/s et dans une taille de particule moyenne de 60 à 100 microns ; et
(f) un rapport de chute de pression sur une durée de vie de distribution de ladite composition provenant dudit conteneur inférieur à 4/1 lorsque ledit conteneur a un espace libre de 25 % en volume et de 2/1 lorsque ledit conteneur a un espace libre de 50 % en volume.

2. L'article de la revendication 1, dans lequel ledit gaz comprimé en est un ou plusieurs parmi l'air, l'azote, le dioxyde de carbone, l'oxyde nitreux, et un gaz inerte.

3. L'article de la revendication 1, dans lequel ledit au moins un ingrédient actif en est un ou plusieurs parmi un parfum, un composé éliminant les odeurs, un insecticide, un antimicrobien, et un désinfectant.

4. L'article de la revendication 1, dans lequel ladite vitesse de pulvérisation va de 1,6 g/s à 2,5 g/s.

5. L'article de la revendication 1, dans lequel ladite taille de particule moyenne va de 60 à 90 microns.

6. L'article de la revendication 1, dans lequel ladite taille de particule moyenne va de 60 à 80 microns.
